# EUROPEAN PATENT APPLICATION

(11) **EP 4 248 869 A1**
(43) Date of publication of application: **27.09.2023**
(21) Application number: 23730693.1
(22) Date of filing: 05.01.2023
(51) Int. Cl.: A61B 5/339, A61B 5/332, A61B 5/352, A61B 5/361, A61B 5/364

(54) **DISPLAY CONTROL DEVICE, DISPLAY CONTROL METHOD, AND PROGRAM**

(30) Priority: 12.01.2022 JP 2022002935
(71) Applicant: Cardio Intelligence Inc., Minato-ku Tokyo 106-0044 (JP)
(72) Inventor: TAKATA, Tomohiro, Tokyo 1060044 (JP); TANIGUCHI, Hirohisa, Tokyo 1060044 (JP); TAMURA, Yuichi, Tokyo 1060044 (JP); TAKECHI, Mineki, Tokyo 1060044 (JP); HATANO, Kaoru, Tokyo 1060044 (JP); MIYUKI, Yusuke, Tokyo 1060044 (JP); OKAMOTO, Katsuro, Tokyo 1060044 (JP); WADA, Akane, Tokyo 1060044 (JP)
(74) Representative: Plougmann Vingtoft a/s
(86) International application number: PCT/JP2023/000046
(87) International publication number: WO 2023/136189

(57) **Abstract**

A display control apparatus 3 according to a first embodiment of the present disclosure includes an acquisition part 331 that acquires a value indicating the electric activity of the heart of a person to be analyzed, and a display control part 335 that causes a display part to display a plurality of points corresponding to a plurality of values measured in a target period, wherein a distance between the predetermined reference point and each of the plurality of points corresponds to the value corresponding to the point, and a direction of a vector from the reference point to each of the plurality of points rotates clockwise or counterclockwise about the reference point in the order of times at which the plurality of values are measured.

## Description

### TECHNICAL FIELD

The present disclosure relates to a display control apparatus, a display control method, and a program for displaying information about electric activity of the heart.

### BACKGROUND OF THE INVENTION

Conventionally, a Holter monitor that can be worn on the body of a person to be analyzed, such as a patient, to produce an electrocardiogram over a long period of time is known (see, Patent Document 1, for example).

### PRIOR ART

### PATENT DOCUMENT

[Patent Document 1] Japanese Unexamined Patent Application Publication No. 2007-195693

### SUMMARY OF INVENTION

### PROBLEMS TO BE SOLVED BY THE INVENTION

Conventionally, by visually confirming change over time of a value indicating electric activity of the heart such as an R-R interval in an electrocardiogram produced by an electrocardiograph, an analyzer such as a doctor determines whether abnormality such as atrial fibrillation occurs in a person to be analyzed. However, since the horizontal axis represents duration and the vertical axis represents electrical excitation of the heart in a graph of the electrocardiogram, it is difficult for the analyzer, who is required to compare waveforms at positions apart from each other on the graph, to intuitively confirm change over time of the value indicating the electric activity of the heart. Therefore, there were cases where it was difficult for the analyzer to confirm change over time of the value indicating the electric activity of the heart without overlooking such a change.

The present disclosure focuses on this point, and its object is to make it easy for an analyzer to confirm change over time of a value indicating electric activity of the heart.

### MEANS FOR SOLVING THE PROBLEMS

A display control apparatus according to a first aspect of the present disclosure includes an acquisition part that acquires a value indicating electric activity of the heart of a person to be analyzed; and a display control part that causes a display part to display a plurality of points corresponding to a plurality of values measured in a target period, wherein a distance between a predetermined reference point and each of the plurality of points corresponds to the value corresponding to the point, and a direction of a vector from the reference point to each of the plurality of points rotates clockwise or counterclockwise about the reference point in the order of times at which the plurality of values are measured.

The display control part may cause the display part to display a polygonal line generated by connecting two adjacent points among the plurality of points with a line.

The display control part may change a display mode of a point corresponding to a value or a line connected to the point, according to the time at which the value is measured.

The display control part may cause the display part to display a plurality of concentric circles arranged at predetermined intervals centered around the reference point together with the plurality of points.

The display control apparatus may further include a selection part that selects, as the target period, a period in which variation of the plurality of values satisfies a predetermined condition from within a period during which measurement of the electric activity is performed.

The display control apparatus may further include a selection part that selects, as the target period, a period during which it is estimated that atrial fibrillation or premature contraction occurs in the person to be analyzed from a period during which a measurement of the electric activity is performed.

The display control apparatus may further include an estimation part that estimates whether atrial fibrillation or premature contraction occurs in the person to be analyzed by inputting a positional relationship among the plurality of points corresponding to the plurality of values of the person to be analyzed to a machine learning model that is machine-learned using (i) a positional relationship among a plurality of points corresponding to a plurality of values in a period in which it is determined that atrial fibrillation or premature contraction is occurring in a person to be learned, and (ii) a positional relationship among a plurality of points corresponding to a plurality of values in a period in which it is determined that atrial fibrillation or premature contraction is not occurring in the person to be learned.

The display control part may cause the display part to display (i) the plurality of points in the target period in which it is estimated that atrial fibrillation or premature contraction occurs in the person to be analyzed and (ii) the plurality of points in the target period in which it is estimated that atrial fibrillation or premature contraction does not occur in the person to be analyzed, in different display modes.

The acquisition part may calculate circularity that becomes smaller as a shape of a polygonal line generated by connecting two adjacent points among the plurality of points is closer to a true circle, and the display control part may cause the display part to display the plurality of points whose circularity is equal to or less than a threshold value and the plurality of points whose circularity is greater than the threshold value in different display modes.

The longer the target period or greater the number of the plurality of points, the smaller the threshold value may be.

The display control part may cause the display part to display the plurality of points in a second period within the target period in a manner such that the plurality of points in the second period are superimposed on the plurality of points in a first period within the target period.

An angle between two points relative to the reference point may be an angle proportional to the value corresponding to one of the two points.

The display control part may cause the display part to sequentially display the points corresponding to the values in response to the values being measured.

The display control part may stop displaying the points corresponding to the values on the display part sequentially if variation of the plurality of values becomes equal to or greater than a predetermined threshold value while the points corresponding to the values are sequentially displayed on the display part.

The display control apparatus may further include: a detection part that detects a tap event indicating that the person to be analyzed tapped a device for measuring the electric activity, on the basis of acceleration measured by a sensor provided in the device, the device being worn by the person to be analyzed; and a selection part that selects, as the target period, a period based on a time at which the tap event is detected from a period during which the electric activity is measured.

The display control apparatus may further include: an estimation part that estimates whether atrial fibrillation occurs in the person to be analyzed on the basis of the electric activity measured during a period based on a time at which the tap event is detected, wherein the display control part causes the display part to display information indicating whether atrial fibrillation estimated by the estimation part occurs in the person to be analyzed.

The detection part may detect the tap event on condition that the sensor measures acceleration equal to or greater than a threshold value which is greater than acceleration generated in the sensor by the electric activity.

A display control method according to a second aspect of the present disclosure is executed by a processor and includes the steps of: acquiring a value indicating electric activity of the heart of a person to be analyzed; and causing a display part to display a plurality of points corresponding to a plurality of values measured in a target period, wherein a distance between a predetermined reference point and each of the plurality of points corresponds to the value corresponding to the point, and a direction of a vector from the reference point to each of the plurality of points rotates clockwise or counterclockwise about the reference point in the order of times at which the plurality of values are measured.

A program according to a third aspect of the present disclosure causes a computer to function as: an acquisition part that acquires a value indicating electric activity of the heart of a person to be analyzed; and a display control part that causes a display part to display a plurality of points corresponding to a plurality of values measured in a target period, wherein a distance between a predetermined reference point and each of the plurality of points corresponds to the value corresponding to the point, and a direction of a vector from the reference point to each of the plurality of points rotates clockwise or counterclockwise about the reference point in the order of times at which the plurality of values are measured.

### EFFECT OF THE INVENTION

According to the present disclosure, an effect that an analyzer can easily confirm change over time of a value indicating electric activity of the heart is achieved.

### BRIEF DESCRIPTION OF THE DRAWINGS

FIG. 1 illustrates an outline of a display control system S according to an embodiment.
FIG. 2 is a block diagram of the display control system S according to the embodiment.
FIG. 3 a schematic diagram illustrating a method in which a detection part detects a tap event.
FIG. 4 is a schematic diagram of an information terminal displaying information indicating a relationship between a tap event and the presence or absence of atrial fibrillation.
FIG. 5 is a schematic diagram illustrating a method in which a selection part selects a second target period.
FIG. 6 is a schematic diagram illustrating a method in which a display control part causes a display part to display a plurality of points corresponding to a plurality of feature values.
FIG. 7 is a schematic diagram for illustrating the shapes of exemplary polygonal figures.
FIG. 8 is a schematic diagram of an information terminal displaying a polygonal figure corresponding to the feature values.
FIG. 9 is a flowchart of a display control method executed by the display control system according to the embodiment.
FIG. 10 is a schematic diagram of an information terminal displaying a polygonal figure corresponding to the feature values.
FIG. 11 is a schematic diagram for illustrating the shape of an exemplary polygonal figure.
FIG. 12 is a schematic diagram for illustrating the shape of an exemplary polygonal figure.
FIG. 13 is a schematic diagram for illustrating the shape of an exemplary polygonal figure.

### DETAILED DESCRIPTION OF THE INVENTION

### [Outline of a display control system S]

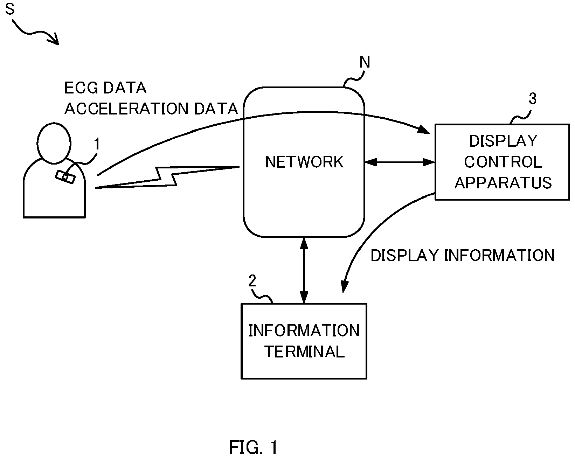

FIG. 1 illustrates an outline of a display control system S according to the present embodiment. The display control system S includes measurement equipment 1, an information terminal 2, and a display control apparatus 3. A plurality of pieces of measurement equipment 1 and a plurality of information terminals 2 may be provided. The display control system S may include equipment such as other servers and terminals.

The measurement equipment 1 is a device that produces an electrocardiogram of the heart of a person to be analyzed. The measurement equipment 1 includes a sensor that measures acceleration applied to the measurement equipment 1. The person to be analyzed is a patient receiving medical care, a subject participant in clinical trials, or the like, for example. The measurement equipment 1 is an electrocardiograph worn by the person to be analyzed, for example. The measurement equipment 1 produces (i) a body surface electrocardiogram on the surface of the body of the person to be analyzed or (ii) an intracardiac electrocardiogram on or within the surface of the heart of the person to be analyzed.

The measurement equipment 1 is a 12-lead electrocardiograph, a Holter monitor, or an insertable cardiac monitor which produces an electrocardiogram of a person to be analyzed by measuring a potential while being worn on the person's wrist, palm, chest, or the like, for example. In the present embodiment, the measurement equipment 1 is a patch electrocardiograph capable of continuously measuring the electrocardiogram of the person to be analyzed living their daily life, by being attached to the chest of the person to be analyzed. The measurement equipment 1 may be another device capable of producing an electrocardiogram, such as a pacemaker, a defibrillator, an intracardiac electrode, or the like.

The measurement equipment 1 transmits electrocardiogram data indicating the produced electrocardiogram and acceleration data indicating the measured acceleration to the display control apparatus 3 via a network N including a wireless communication line. The electrocardiogram data and the acceleration data measured by the measurement equipment 1 may be sent to the display control apparatus 3 using a storage medium without passing through the network N, for example.

The information terminal 2 is a computer used by an analyzer such as a doctor, a medical worker, or the like. The information terminal 2 includes a display part such as a liquid crystal display capable of displaying information received from the display control apparatus 3, for example.

The display control apparatus 3 is a computer that performs control to display information about electric activity of the heart on the display part. The display control apparatus 3 transmits display information for displaying the information about electric activity of the heart to the information terminal 2 on the basis of the electrocardiogram data and the acceleration data received from the measurement equipment 1, for example.

An outline of processing executed by the display control system S according to the present embodiment will be described below. The measurement equipment 1 produces the electrocardiogram of the person to be analyzed wearing the measurement equipment 1, and also measures the acceleration applied to the measurement equipment 1. The measurement equipment 1 transmits electrocardiogram data indicating the produced electrocardiogram and acceleration data indicating the measured acceleration to the display control apparatus 3.

The display control apparatus 3 acquires the electrocardiogram data and the acceleration data transmitted by the measurement equipment 1. The display control apparatus 3 acquires a feature value that is a value indicating electric activity of the heart of a person to be analyzed from the electrocardiogram data. The feature value is a value extracted from the electrocardiogram data and is an R-R interval, for example.

Further, the display control apparatus 3 detects a tap event indicating that the person to be analyzed tapped the device, on the basis of the received acceleration data. The display control apparatus 3 detects the tap event on condition that a peak of acceleration occurred three times or more within 10 seconds, for example.

The display control apparatus 3 selects a predetermined period based on the time at which the tap event is detected as a target period used for displaying the feature value, for example. The display control apparatus 3 generates display information for displaying a plurality of points corresponding to a plurality of feature values measured in the target period on the display part of the information terminal 2, and transmits the display information to the information terminal 2.

Here, the display control apparatus 3 causes the display part to display the plurality of points corresponding to the plurality of feature values such that they become vertices of a polygonal figure with respect to a predetermined reference point. The polygonal figure is a figure formed by a closed polygonal line or an open polygonal line. The display control apparatus 3 may cause the display part to display only the vertices of the polygonal figure. In the displayed polygonal figure, a distance between the reference point and each of the plurality of points corresponds to the feature value corresponding to said point.

In making a diagnosis of arrhythmia, evaluating responses to stimuli in an electrophysiology study, or the like, it is necessary to confirm whether electric activity of the heart is periodical or irregular. Conventionally, whether the electric activity of the heart is irregular or not has been confirmed using an electrocardiogram in which the horizontal axis represents duration and the vertical axis represents electrical excitation of the heart, or a trend graph in which the horizontal axis represents duration and the vertical axis represents heart rate. However, from such an electrocardiogram or trend graph, it was not easy for an analyzer to intuitively grasp whether the electric activity of the heart is irregular.

On the other hand, according to the display control system S of the present embodiment, the analyzer can grasp change over time of the feature values in the target period as the shape of a polygonal figure in the information terminal 2. For example, the shape of the polygonal figure becomes close to a true circle in a normal state (sinus rhythm), and unevenness of the overall shape of the polygonal figure increases in a state in which atrial fibrillation occurs in a person to be analyzed. Further, in a situation in which premature contraction occurs in a person to be analyzed, unevenness of only a part of the shape of the polygonal figure increases, for example. As a result, the display control system S can make it easier for the analyzer to confirm change over time of the value indicating the electric activity of the heart.

### [Configuration of the display control system S]

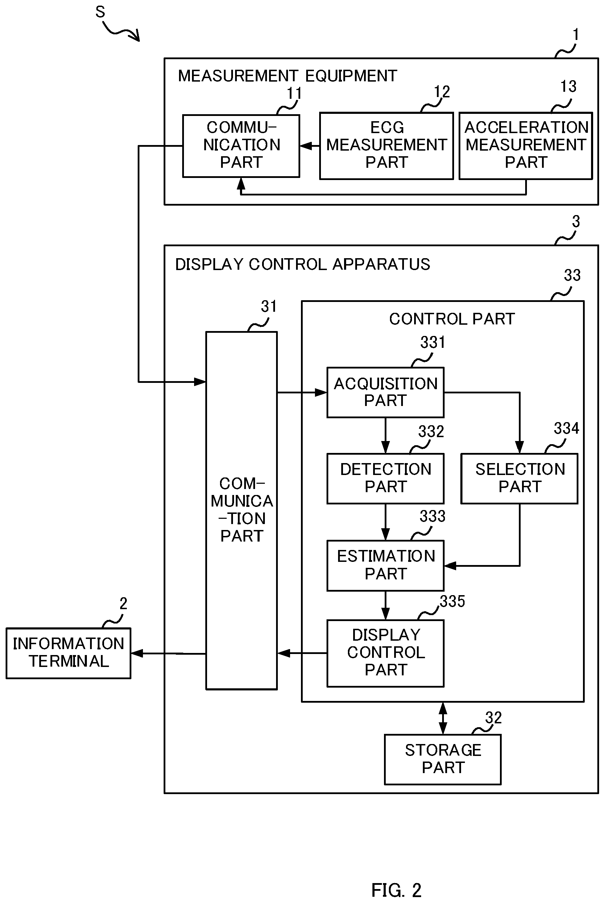

FIG. 2 is a block diagram of the display control system S according to the present embodiment. In FIG. 2, arrows indicate main data flows, and there may be data flows other than those shown in FIG. 2. In FIG. 2, each block indicates a configuration of a function unit, not a configuration of a hardware (device) unit. As such, the blocks shown in FIG. 2 may be implemented in a single device or separately in a plurality of devices. The transfer of data between the blocks may be performed via any means, such as a data bus, a network, a portable storage medium, or the like.

The measurement equipment 1 includes a communication part 11, an electrocardiogram measurement part 12, and an acceleration measurement part 13. The measurement equipment 1 may be configured by connecting two or more physically separated devices in a wired or wireless manner.

The communication part 11 has a communication controller for transmitting and receiving data to and from the display control apparatus 3 via the network N. The communication part 11 transmits data output from the electrocardiogram measurement part 12 and the acceleration measurement part 13 to the display control apparatus 3 via the network N.

The electrocardiogram measurement part 12 includes an electrode disposed on or inside the body of the person to be analyzed, and a circuit for measuring a potential via the electrode, and outputs electrocardiogram data indicating an electrocardiogram produced from the measured potential to the communication part 11, for example. The acceleration measurement part 13 includes an acceleration sensor that measures acceleration applied to the measurement equipment 1, and outputs acceleration data indicating the measured acceleration to the communication part 11, for example.

The display control apparatus 3 has a communication part 31, a storage part 32, and a control part 33. The display control apparatus 3 may be configured by connecting two or more physically separated devices in a wired or wireless manner. Further, the display control apparatus 3 may be formed by a cloud which is a collection of computer resources.

The communication part 31 has a communication controller for transmitting and receiving data between the measurement equipment 1 and the information terminal 2 via the network N. The communication part 31 notifies the control part 33 of data received from the measurement equipment 1 via the network N. The communication part 31 transmits data output from the control part 33 to the information terminal 2 via the network N.

The storage part 32 is a storage medium including a read only memory (ROM), a random access memory (RAM), a hard disk drive, and the like. The storage part 32 stores in advance a program to be executed by the control part 33. The storage part 32 may be provided outside the display control apparatus 3, and in such a case, the storage part 32 may exchange data with the control part 33 via a network.

The control part 33 has an acquisition part 331, a detection part 332, an estimation part 333, a selection part 334, and a display control part 335. The control part 33 is a processor such as a central processing unit (CPU) and functions as the acquisition part 331, the detection part 332, the estimation part 333, the selection part 334, and the display control part 335 by executing the program stored in the storage part 32. At least some of the functions of the control part 33 may be executed by an electric circuit. Further, at least some of the functions of the control part 33 may be executed by the control part 33 executing a program executed via a network.

Hereinafter, a display control method executed by the display control system S according to the present embodiment will be described in detail. A person to be analyzed wears the measurement equipment 1. In the measurement equipment 1, the electrocardiogram measurement part 12 produces an electrocardiogram of the person to be analyzed wearing the measurement equipment 1. The acceleration measurement part 13 measures acceleration applied to the measurement equipment 1. The communication part 11 transmits electrocardiogram data indicating the electrocardiogram produced by the electrocardiogram measurement part 12 and acceleration data indicating the acceleration measured by the acceleration measurement part 13 to the display control apparatus 3.

The communication part 11 sequentially transmits the electrocardiogram data and the acceleration data to the display control apparatus 3, or collectively transmits the electrocardiogram data and the acceleration data for a predetermined time period (for example, 24 hours) to the display control apparatus 3. The communication part 11 transmits the electrocardiogram data and the acceleration data to the display control apparatus 3 at the same time or at different timings.

The display control apparatus 3 executes at least one of (1) a process for displaying information about a tap event on the display part or (2) a process for displaying a polygonal figure corresponding to values indicating electric activity of the heart on the display part. The display control apparatus 3 may execute these two processes in parallel, or may execute one of these processes before executing the other process.

First, the process for causing the display control apparatus 3 to display the information about the tap event on the display part will be described. In the display control apparatus 3, the acquisition part 331 acquires the acceleration data transmitted from the measurement equipment 1 via the communication part 31. The person to be analyzed is instructed to tap the measurement equipment 1 a predetermined number of times when symptoms of atrial fibrillation occur and when he/she has a meal, exercises, or the like. The detection part 332 detects, on the basis of the acceleration data acquired by the acquisition part 331, a tap event indicating that the person to be analyzed tapped the device. The detection part 332 detects the tap event on condition that a peak of acceleration occurred a predetermined number of times or more within a predetermined time period in the acceleration data, for example.

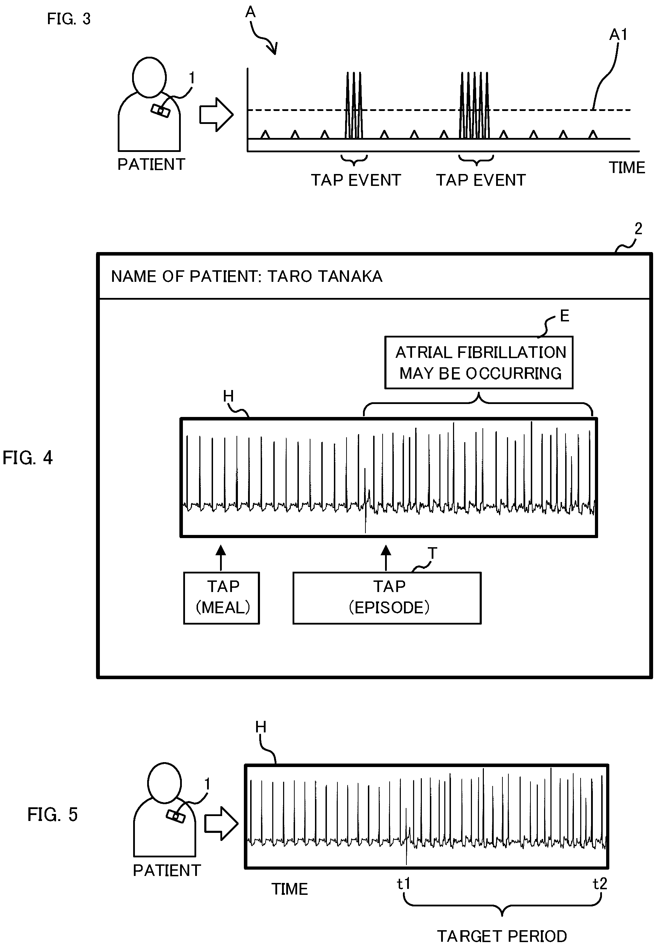

FIG. 3 is a schematic diagram illustrating a method in which the detection part 332 detects a tap event. FIG. 3 shows a graph of acceleration indicated by acceleration data A. In the graph of FIG. 3, the horizontal axis represents measurement time and the vertical axis represents acceleration.

The detection part 332 identifies a peak of acceleration that is equal to or greater than a predetermined threshold value A1 in the graph of acceleration. The threshold value A1 is a value greater than acceleration generated in the measurement equipment 1 by electric activity of the heart such as a heartbeat or the like, for example. The storage part 32 stores in advance the threshold value A1 that is greater than acceleration that occurs in the measurement equipment 1 due to a general heartbeat or the like, for example. In addition, the detection part 332 may determine, as the threshold value A1, a normal acceleration that occurs in the measurement equipment 1 due to the heartbeat or the like of the person to be analyzed on the basis of acceleration data obtained when the person to be analyzed wears the measurement equipment 1 before a period in which the analysis is to be performed (for example, at the time of calibration of the measurement equipment 1).

If peaks are identified a predetermined number of times or more (e.g., three or more times) within a predetermined time period (e.g., within 10 seconds) in the graph of acceleration, the detection part 332 detects these peaks occurring the predetermined number of times or more as the tap event. For example, the detection part 332 identifies an average time value of a plurality of peaks corresponding to the detected tap event as a time at which the tap event is detected.

By comparing the acceleration indicated by the acceleration data to the threshold value A1, the display control apparatus 3 can detect acceleration caused by the person to be analyzed tapping the measurement equipment 1 in distinction from acceleration due to the heartbeat or the like of the person to be analyzed and acceleration caused by the person to be analyzed becoming unable to stand when atrial fibrillation occurs.

Further, the detection part 332 may determine the type of the tap event on the basis of the number of times that a plurality of peaks corresponding to the detected tap event occur. The person to be analyzed is instructed to tap the measurement equipment 1 three times in a row when symptoms of atrial fibrillation such as palpitations occur and to tap the measurement equipment 1 five times in a row when he/she has a meal or exercises.

The storage part 32 stores in advance information in which (i) the number of times the person to be analyzed taps the measurement equipment 1 (that is, the number of times peaks of acceleration occur within the predetermined time period) and (ii) the type of tap event are associated with each other. On the basis of this information stored in the storage part 32, the detection part 332 determines the types of the tap events associated with respective numbers of times of each of one or more tap events being detected. In this way, the person to be analyzed can notify the types of the tap events to the display control apparatus 3 simply by changing the way he/she taps the measurement equipment 1. The display control apparatus 3 can make it easier for the analyzer to analyze the relationship between the electrocardiogram and the type of tap event by causing the display part to display the determined type of tap event.

The detection part 332 causes the storage part 32 to store tap event information in which (i) a time at which each of one or more tap events is detected and (ii) the type of the tap event are associated with each other.

The estimation part 333 estimates whether atrial fibrillation occurs in the person to be analyzed on the basis of electric activity measured during a first target period based on the time at which a tap event is detected by the detection part 332. The first target period is a period of a predetermined length (5 minutes, 10 minutes, or the like) including the time at which the tap event is detected, for example. The first target period may be a period of a predetermined length at least one of before or after the time at which the tap event is detected.

The storage part 32 stores in advance a first machine learning model that can classify input electrocardiogram data generated by learning on the basis of learning electrocardiogram data, used as training data, into (i) electrocardiogram data in which atrial fibrillation occurs and (ii) electrocardiogram data in which atrial fibrillation does not occur, for example. An internal configuration of the first machine learning model is any desired configuration, and it includes a convolutional neural network (CNN), for example.

The estimation part 333 inputs electrocardiogram data of the first target period within the electrocardiogram data acquired by the acquisition part 331 to the first machine learning model stored in the storage part 32. The estimation part 333 estimates whether atrial fibrillation occurs in the person to be analyzed in the first target period on the basis of a classification result output from the first machine learning model.

Further, the estimation part 333 may input each of a plurality of pieces of electrocardiogram data generated by dividing the entire period of the electrocardiogram data acquired by the acquisition part 331 into predetermined lengths (5 minutes, 10 minutes, or the like) to the first machine learning model stored in the storage part 32. In this case, the estimation part 333 estimates whether atrial fibrillation occurs in the person to be analyzed in the first target period by extracting classification results in the first target period from a classification result of the entire period.

When it is estimated that atrial fibrillation occurs in the person to be analyzed in the first target period, the estimation part 333 further determines whether fibrillation waves (also referred to as f waves) are clear in the electrocardiogram data of the first target period.

The storage part 32 stores in advance a second machine learning model that can classify input electrocardiogram data generated by learning on the basis of learning electrocardiogram data, used as training data, into (i) electrocardiogram data whose fibrillation waves are clear and (ii) electrocardiogram data whose fibrillation waves are unclear, for example. An internal configuration of the second machine learning model is any desired configuration, and it includes a CNN, for example.

The estimation part 333 inputs each of a plurality of pieces of electrocardiogram data generated by dividing the electrocardiogram data of the first target period into predetermined lengths (30 seconds, 1 minute, or the like) to the second machine learning model stored in the storage part 32. The estimation part 333 identifies a portion with clear fibrillation waves within the electrocardiogram data of the first target period on the basis of a classification result output from the second machine learning model. Further, the estimation part 333 may identify a portion with little noise in the electrocardiogram data of the first target period, if electrocardiogram data whose fibrillation waves are clear is not identified in the electrocardiogram data of the first target period. For example, the estimation part 333 may select, as the portion with little noise, a waveform having a low peak level of a high-frequency component or a low-frequency component using a Fourier transform, or may create a predictive model for electrocardiogram data using a moving average method or an AR model to select a portion with a small amount of deviation from the predictive model, as the portion with little noise. Further, the estimation part 333 may identify the portion with little noise by using a machine learning model generated by machine-learning electrocardiogram data determined to have little noise and electrocardiogram data determined to have much noise by a laboratory technician or a doctor.

The display control part 335 generates display information for displaying information indicating a relationship between the tap event detected by the detection part 332 and the presence or absence of atrial fibrillation estimated by the estimation part 333, together with the electrocardiogram data of the first target period, on the display part of the information terminal 2. The display information includes the electrocardiogram data of the first target period, tap event information about a tap event detected by the detection part 332, and estimation information about the presence or absence of atrial fibrillation estimated by the estimation part 333, for example.

The display information may include only the portion the electrocardiogram data of the first target period with clear fibrillation waves identified by the estimation part 333. In addition, the display information may include only the portion of the electrocardiogram data of the first target period with little noise identified by the estimation part 333, if the electrocardiogram data whose fibrillation waves are clear is not identified in the electrocardiogram data of the first target period. By doing this, the display control apparatus 3 causes the display part to display only a portion that is likely to contribute to the analysis in a period in which it is estimated that atrial fibrillation occurs, thereby making it easy for the analyzer to analyze the electrocardiogram data.

The display control part 335 transmits the generated display information to the information terminal 2. According to the display information received from the display control apparatus 3, the information terminal 2 displays the information indicating the relationship between the tap event detected by the detection part 332 and the presence or absence of atrial fibrillation estimated by the estimation part 333, together with the electrocardiogram data of the first target period, on the display part.

FIG. 4 is a schematic diagram of the information terminal 2 displaying information indicating a relationship between a tap event and the presence or absence of atrial fibrillation. In the example of FIG. 4, the display control part 335 displays tap information T and estimation information E on the display part of the information terminal 2, together with electrocardiogram data H of the first target period.

The tap information T includes information identifying a time at which each of one or more tap events is detected (for example, an arrow indicating the time on the electrocardiogram data H) within the electrocardiogram data H of the first target period, and a type of the tap event, for example. The estimation information E includes a character string or a symbol representing whether it is estimated that atrial fibrillation occurs in the person to be analyzed in the first target period, for example. Further, the estimation information E indicates the portion with clear fibrillation waves within the electrocardiogram data H of the first target period.

In examining or making a diagnosis of arrhythmia such as atrial fibrillation, it is important to confirm whether the characteristics specific to arrhythmia appear in a waveform of the electrocardiogram at a timing when the person to be analyzed has symptoms such as palpitations or the like, or at a timing when the person to be analyzed performs a specific action such as having a meal, exercising, or the like. The display control apparatus 3 causes the display part to display the information indicating the relationship between the tap event detected by the detection part 332 and the presence or absence of atrial fibrillation estimated by the estimation part 333. In this manner, the display control apparatus 3 can make it easier to analyze a waveform of the electrocardiogram at a timing when the analyzer felt symptoms of arrhythmia or the like. Further, by displaying the type of the tap event on the display part, the display control apparatus 3 makes it easy for the analyzer to analyze what kind of event affected the waveform of the electrocardiogram.

Secondly, a process for displaying a polygonal figure corresponding to values indicating electric activity of the heart on the display part with the display control apparatus 3 will be described. In the display control apparatus 3, the acquisition part 331 acquires the electrocardiogram data transmitted by the measurement equipment 1 via the communication part 31.

The acquisition part 331 acquires a feature value that is a value indicating electric activity of the heart of the person to be analyzed from the electrocardiogram data. The feature value is a value extracted from the electrocardiogram data. The acquisition part 331 identifies a measurement time on the electrocardiogram data corresponding to the feature value as a time at which the feature value is measured.

The feature value is an RR interval (R-R interval) indicating a time between peaks of a QRS wave (R wave) in an electrocardiogram, or the heart rate per unit time, for example. The heart rate per unit time corresponds to the inverse of the R-R interval. Further, the feature value may be programmed stimulation given from outside during a cardiac electrophysiological examination or a response to the programmed stimulation, and is a V-V interval, a V-A interval, a His-V interval, an interval between a potential of a catheter electrode and any response waveform, or the like of an inner electrocardiogram, for example. Further, the feature value may be an electrode catheter interval at the time of supraventricular arrhythmia ablation, the earliest stage of anatomical electromotive force at the time of atrial fibrillation (AF) ablation, or a value for a purpose of making a diagnosis or treating other diseases, and is the earliest stage of electromotive force at the time of premature ventricular contraction (PVC) or a distance between a mid-diastolic potential (MDP) of ventricular tachycardia (VT) and a QRS wave, for example. The acquisition part 331 acquires any one of the R-R interval, the heart rate per unit time, the V-V interval, and the V-A interval as the feature value by performing a known waveform analysis process (e.g., wavelet transformation) on the electrocardiogram data.

The selection part 334 selects at least a part of the period during which the measurement of the electric activity of the heart is performed on the person to be analyzed (for example, the entire period of the electrocardiogram data), as a second target period during which the feature value is to be displayed.

FIG. 5 is a schematic diagram illustrating a method in which the selection part 334 selects the second target period. FIG. 5 shows a graph of the electrocardiogram indicated by the electrocardiogram data H. In the graph of FIG. 5, the horizontal axis represents measurement time and the vertical axis represents a potential of the electrocardiogram.

For example, the selection part 334 selects, as the second target period, a period in which variation of a plurality of feature values satisfies a predetermined condition, from within the period during which the measurement of the electric activity of the heart is performed. In this case, the selection part 334 calculates the variation for each of a plurality of periods generated by dividing the entire period of the electrocardiogram data into a predetermined length (5 minutes, 10 minutes, or the like).

The selection part 334 calculates a statistical value such as dispersion or standard deviation of a plurality of feature values measured in one period as the variation of the plurality of feature values in said period, for example. Further, the selection part 334 may calculate a value corresponding to a difference from a mode value or a median value of the plurality of feature values measured in one period, as the variation of the plurality of feature values in said period. For example, the selection part 334 selects, as the second target period, one or more periods in which the calculated variation is equal to or greater than a predetermined threshold from within the entire period of the electrocardiogram data.

In the example of FIG. 5, the selection part 334 selects, as the second target period, a period from a first time t1 to a second time t2 in which the variation of the plurality of feature values is equal to or greater than the predetermined threshold. By doing this, the display control apparatus 3 can extract a portion with a large variation among the plurality of feature values, which is important for checking whether atrial fibrillation occurs, in the electrocardiogram data, as a display subject.

Further, the selection part 334 may select, as the second target period, one or more periods designated by the analyzer in the information terminal 2 from within the period during which the measurement of the electric activity of the heart is performed.

The selection part 334 may select the second target period on the basis of the tap event detected by the detection part 332. In this case, the selection part 334 selects, as the second target period, a period of a predetermined length (5 minutes, 10 minutes, or the like) including a time at which a tap event is detected by the detection part 332, from within the period during which the measurement of the electric activity of the heart is performed, for example. Further, the selection part 334 may select, as the second target period, a period of a predetermined length at least one of before or after the time at which the tap event is detected. In this manner, the display control apparatus 3 can extract a portion close to a timing at which the analyzer felt that there were symptoms of arrhythmia or the like in the electrocardiogram data as the display subject, for example.

Further, the selection part 334 may select the second target period on the basis of the presence or absence of atrial fibrillation estimated by the estimation part 333. In this case, the selection part 334 selects, as the second target period, a period in which the estimation part 333 estimated that atrial fibrillation occurs from within the period during which the measurement of the electric activity of the heart is performed, for example. As a result, the display control apparatus 3 can extract a portion in which it is estimated that atrial fibrillation occurs in the electrocardiogram data, as the display subject.

The estimation part 333 estimates whether atrial fibrillation occurs in the person to be analyzed on the basis of the electric activity measured during the second target period selected by the selection part 334. If the selection part 334 selects the period in which the estimation part 333 has estimated that atrial fibrillation occurs as the second target period, the estimation part 333 does not need to estimate the presence or absence of atrial fibrillation again.

The estimation part 333 inputs electrocardiogram data of the second target period within the electrocardiogram data acquired by the acquisition part 331 to the first machine learning model stored in the storage part 32. The estimation part 333 estimates whether atrial fibrillation occurs in the person to be analyzed in the second target period on the basis of the classification result output from the first machine learning model.

Further, the estimation part 333 may input each of a plurality of pieces of electrocardiogram data generated by dividing the entire period of the electrocardiogram data acquired by the acquisition part 331 into predetermined lengths (5 minutes, 10 minutes, or the like) to the first machine learning model stored in the storage part 32. In this case, the estimation part 333 estimates whether atrial fibrillation occurs in the person to be analyzed in the second target period by extracting classification results in the second target period from a classification result of the entire period.

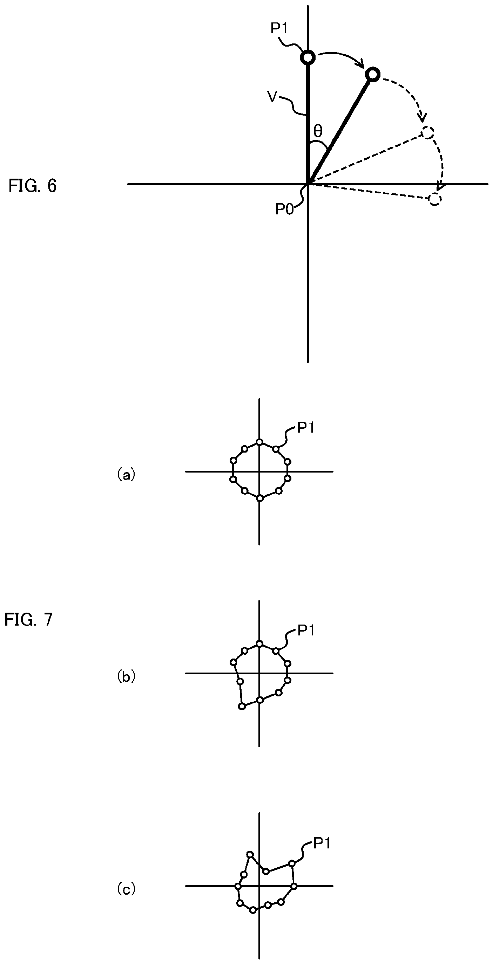

The display control part 335 generates display information for causing the display part of the information terminal 2 to display a plurality of points corresponding to a plurality of feature values measured in the second target period, as a polygonal figure. FIG. 6 is a schematic diagram illustrating a method in which the display control part 335 causes the display part to display a plurality of points P1 corresponding to a plurality of feature values.

The display control part 335 arranges the plurality of points P1 corresponding to the plurality of feature values on a two-dimensional plane relative to a predetermined reference point P0 (origin), according to the following rules.
(1) A distance between the reference point P0 and each of the plurality of points P1 (i.e., the magnitude of a vector V) is a value corresponding to the feature value corresponding to said point P1.
(2) A direction of the vector V from the reference point P0 to each of the plurality of points P1 rotates at an angle θ clockwise or counterclockwise about the reference point P0 in the order of the times at which the plurality of feature values are measured.

By doing this, the plurality of points P1 corresponding to the plurality of feature values are arranged such that they become vertices of the polygonal figure relative to the reference point P0. The entire second target period may be represented with one cycle (360 degrees) of a polygonal figure. Further, a plurality of periods constituting the second target period may be represented with multiple cycles (720 degrees, 1080 degrees, or the like) of a polygonal figure.

If a period corresponding to the one cycle of the polygonal figure is too long, variation in the feature value due to atrial fibrillation affects only a part of the shape of the polygonal figure, and therefore it is difficult for the analyzer to confirm the presence or absence of atrial fibrillation on the basis of the shape of the polygonal figure. For this reason, the period corresponding to the one cycle of the polygonal figure, that is, the length of the period corresponding to the one cycle is a value within 5 seconds or more and 5 minutes or less. More preferably, the length of the period corresponding to the one cycle is within 10 seconds or more and 1 minute or less, for example. By doing this, the variation of the feature value due to atrial fibrillation can be easily reflected in the shape of the entire polygonal figure, and therefore the display control apparatus 3 can make it easy for the analyzer to confirm the presence or absence of atrial fibrillation on the basis of the shape of the polygonal figure.

The distance between the reference point P0 and the point P1 is a value obtained by multiplying the feature value corresponding to the point P1 by a predetermined coefficient, for example. Thus, the display control apparatus 3 can express the magnitudes of the plurality of feature values as unevenness of the polygonal figure.

An angle θ is a value obtained by dividing 2π (360 degrees) by the number of vertices of one cycle of the polygonal figure, for example. The number of vertices of one cycle of the polygonal figure is set in the storage part 32. The angle θ may be an angle proportional to a feature value corresponding to one of two adjacent points. In this case, the angle θ is calculated by an equation of R-R interval × 2π/ (length of the period corresponding to the one cycle), for example. The length of the period corresponding to the one cycle is set in the storage part 32. Thus, the display control apparatus 3 can adjust the angle between the vertices according to the magnitude of the feature value.

The feature value such as the R-R interval, the heart rate per unit time, the V-V interval, the V-A interval, or the like is one of the criteria for determining atrial fibrillation. In a state in which atrial fibrillation occurs in the person to be analyzed, irregularity that is not in the normal state (sinus rhythm) appears in the feature value. Further, even in the state in which premature contraction, which is generally said to be higher in safety than atrial fibrillation, occurs in the person to be analyzed, irregularity may appear in the feature value.

However, conventionally, it was not easy for the analyzer such as a doctor or the like to grasp irregularity in the feature value from the electrocardiogram data whose horizontal axis represents duration and vertical axis represents electrical excitation of the heart without overlooking an irregularity, and to distinguish between the normal state, the state in which atrial fibrillation occurs, and the state in which premature contraction occurs. On the other hand, the display control apparatus 3 makes it easy for the analyzer to intuitively grasp the irregularity in the feature values by displaying the polygonal figure corresponding to the plurality of feature values on the display part.

FIGS. 7A, 7B, and 7C are each a schematic diagram for illustrating the shape of an exemplary polygonal figure. FIG. 7A shows a polygonal figure of a normal state (sinus rhythm). FIG. 7B shows a polygonal figure of a state in which premature contraction occurs in the person to be analyzed. FIG. 7C shows a polygonal figure of a state in which atrial fibrillation occurs in the person to be analyzed.

In the normal state, as exemplified in FIG. 7A, the polygonal figure has a shape approximate to a true circle. In the state in which premature contraction occurs in the person to be analyzed, as exemplified in FIG. 7B, the polygonal figure has large unevenness in one portion and a shape approximate to an arc in the other portion. In the state in which atrial fibrillation occurs in the person to be analyzed, as exemplified in FIG. 7C, the polygonal figure has a shape having large unevenness overall.

In FIG. 7A, FIG. 7B, and FIG. 7C, the irregularity of the feature values appears in the shape of the polygonal figure formed by connecting the plurality of points P1 with a closed polygonal line. In addition, the irregularity of the feature values similarly appears in the shape of the polygonal figure formed by connecting the plurality of points P1 with an unclosed polygonal line. Similarly, the irregularity of the feature values appears in the arrangement of the plurality of points P1 which are the vertices of the polygonal figure.

By looking at the shape of the polygonal figure corresponding to the plurality of feature values, the analyzer intuitively grasps the irregularity of the feature values, and can easily distinguish between the normal state, the state in which atrial fibrillation occurs, and the state in which premature contraction occurs.

The acquisition part 331 may calculate circularity indicating how close the shape of the polygonal figure is to a true circle. For example, the acquisition part 331 generates a polygonal figure that is a polygonal line generated by connecting two adjacent points P1 among the plurality of points P1 with a line. The acquisition part 331 calculates outer diameters D at a plurality of places in the generated polygonal figure, and identifies the maximum outer diameter Dmax and the minimum outer diameter Dmin. Then, the acquisition part 331 calculates a value of (Dmax - Dmin) / 2 as the circularity. The calculated circularity becomes a smaller value as it is closer to a true circle (i.e., as it is similar to a true circle), and becomes a larger value as it is farther from a true circle (i.e., as it is not similar to a true circle).

As will be described later, by changing a display mode of the polygonal figure according to the calculated circularity, the display control part 335 can provide the analyzer with an objective indicator indicating whether the shape of the polygonal figure is close to a true circle. Not limited to the specific values shown here, the acquisition part 331 may obtain, as the circularity, other values indicating how close the shape of a specific polygonal figure is to a true circle.

The estimation part 333 may estimate whether atrial fibrillation or premature contraction occurs in the person to be analyzed in the second target period by applying a machine learning process to the shape of the polygonal figure. In this case, the storage part 32 stores in advance a third machine learning model which is generated by learning (i) a positional relationship among a plurality of points P1 corresponding to a plurality of values in a period in which it is determined that atrial fibrillation is occurring in a person to be learned, (ii) a positional relationship among a plurality of points P1 corresponding to a plurality of values in a period in which it is determined that premature contraction is occurring in the person to be learned, and (iii) a positional relationship among a plurality of points P1 corresponding to a plurality of values in a period in which it is determined that atrial fibrillation or premature contraction is not occurring in the person to be learned, as training data, for example. The third machine learning model can classify the input electrocardiogram data into (i) electrocardiogram data in which atrial fibrillation occurs, (ii) electrocardiogram data in which premature contraction occurs, and (iii) electrocardiogram data in which neither atrial fibrillation nor premature contraction occurs. An internal configuration of the third machine learning model is any desired configuration, and it includes a CNN, for example.

The estimation part 333 inputs the positional relationship (for example, coordinates of each of the plurality of points P1) among the plurality of points P1 arranged by the display control part 335 to the third machine learning model stored in the storage part 32. The estimation part 333 estimates whether atrial fibrillation occurs in the person to be analyzed in the second target period and whether premature contraction occurs in the person to be analyzed, on the basis of a classification result output from the third machine learning model. As will be described later, by causing the display part to display information indicating whether atrial fibrillation or premature contraction estimated by the estimation part 333 occurs, the display control part 335 can provide the analyzer with an estimation result obtained by machine learning using the shape of the polygonal figure.

The display control part 335 generates display information including the electrocardiogram data of the second target period and information for displaying a polygonal figure, for example. The information for displaying the polygonal figure is coordinates of each of a plurality of points or an image of a plurality of points arranged in a two-dimensional plane, for example. Further, the display information may include at least one of (i) the circularity acquired by the acquisition part 331 or (ii) the information indicating whether atrial fibrillation or premature contraction estimated by the estimation part 333 occurs.

The display control part 335 transmits the generated display information to the information terminal 2. In accordance with the display information received from the display control apparatus 3, the information terminal 2 displays a polygonal figure corresponding to the feature values on the display part.

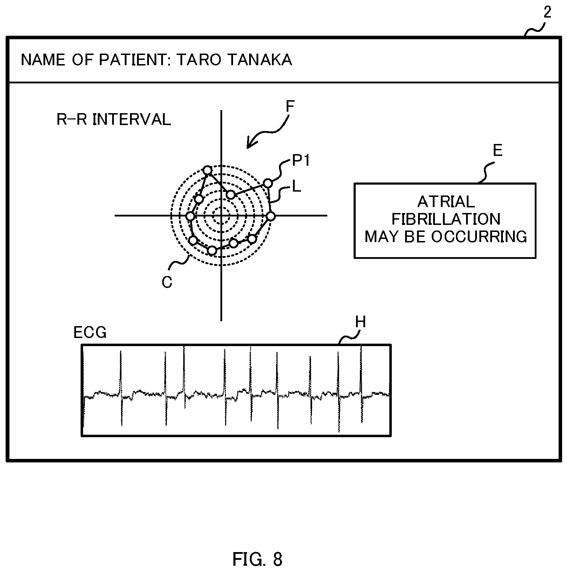

FIG. 8 is a schematic diagram of the information terminal 2 displaying the polygonal figure corresponding to the feature values. In the example of FIG. 8, the display control part 335 causes a polygonal figure F and the estimation information E to be displayed on the display part of the information terminal 2, together with an electrocardiogram data H of the second target period. When a plurality of periods constituting the second target period are represented with multiple cycles of the polygonal figure F, the display control part 335 may cause the display part to display a polygonal figure F of a second period within the second target period superimposed on a polygonal figure F of a first period within the second target period. For example, the first period corresponds to a period corresponding to 360 degrees on the two-dimensional plane, and the second period corresponds to a period corresponding to the next 360 degrees on the two-dimensional plane. Further, the display control part 335 may cause a plurality of polygonal figures F to be displayed in different areas on the display part.

The polygonal figure F includes a plurality of points P1 corresponding to a plurality of feature values, and a polygonal line L generated by connecting two adjacent points among the plurality of points P1 with a line. The polygonal figure F may include only the plurality of points P1 without including the polygonal line L.

The display control part 335 may cause the display part to display a plurality of points P1 in the second target period all at once. Further, the display control part 335 may cause the display part to display the plurality of points P1 in the second target period one by one in the order of the times at which said points P1 are measured.

For example, the display control part 335 generates the polygonal line L by connecting two points P1 from among the plurality of points P1 in the order of the times at which the feature values are measured. Further, the display control part 335 may connect a point P1 which is measured at the earliest time and a point P1 which is measured at the latest time among the plurality of points P1 with a line. Further, the display control part 335 may generate the polygonal line L by connecting two points P1 in the order of positions (coordinates) close to each other among the plurality of points P1. The polygonal line L may be generated by the information terminal 2 instead of the display control part 335 of the display control apparatus 3. By causing change over time of the feature values to be displayed as the shape of the polygonal figure in this manner, the display control apparatus 3 can make it easier for the analyzer to confirm change over time of the feature values.

The polygonal figure F is displayed in a manner such that it is superimposed on a plurality of concentric circles C arranged at predetermined intervals and centered around the reference point P0 used for arranging the plurality of points P1. In this way, the display control apparatus 3 can make it easier for the analyzer to determine whether the shape of the polygonal figure F is close to a true circle.

The estimation information E includes a character string or a symbol indicating whether it is estimated by the estimation part 333 that atrial fibrillation or premature contraction occurs in the person to be analyzed in the second target period, for example. The estimation information E may include information indicating the circularity acquired by the acquisition part 331. The information indicating the circularity includes a character string or a symbol indicating that the shape of the polygonal figure F is close to a true circle when the circularity is equal to or less than a predetermined threshold value, and a character string or a symbol indicating that the shape of the polygonal figure F is far from a true circle when the circularity is greater than the predetermined threshold value, for example.

The display control part 335 may cause the display part to display the plurality of points P1 whose circularity, acquired by the acquisition part 331, is equal to or less than the predetermined threshold value in a display mode different from that of the plurality of points P1 whose circularity is greater than the predetermined threshold value. The display control part 335 changes a color, pattern, or size of the plurality of points P1 or changes a color, type, or thickness of a line connected to each of the plurality of points P1 depending on whether (i) the circularity is equal to or less than the predetermined threshold value or (ii) the circularity is greater than the predetermined threshold value, for example. By doing this, the display control apparatus 3 can make it easier to grasp whether the shape of the polygonal figure F is close to a true circle.

The display control part 335 may cause the display part to display the plurality of points P1 in a case where it is estimated that atrial fibrillation or premature contraction occurs in the person to be analyzed on the basis of the estimation result of the estimation part 333 in a display mode different from that of the plurality of points P1 in a case where it is not estimated that atrial fibrillation or premature contraction occurs in the person to be analyzed. The display control part 335 changes a color, pattern, or size of the plurality of points P1 or changes a color, line type, or thickness of a line that connects the plurality of points P1 depending on a case where it is estimated that atrial fibrillation occurs, a case where it is estimated that premature contraction occurs, and a case where it is estimated that neither atrial fibrillation nor premature contraction is occurring, for example. By doing this, the display control apparatus 3 can make it easier to grasp the estimation result obtained by machine learning that uses the shape of the polygonal figure F.

### [Flowchart of the display control method]

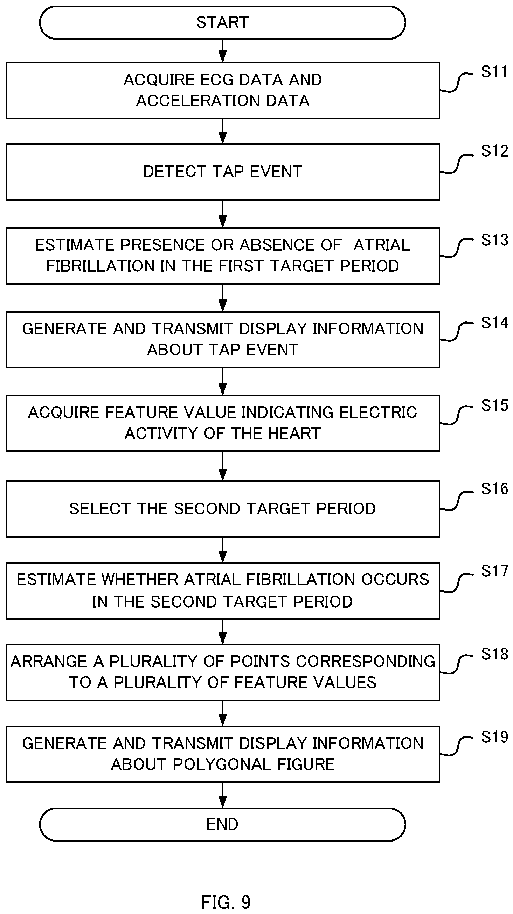

FIG. 9 is a flowchart of a display control method executed by the display control system S according to the present embodiment. In the display control apparatus 3, the acquisition part 331 acquires electrocardiogram data and acceleration data transmitted by the measurement equipment 1 via the communication part 31 (S11).

On the basis of the acceleration data acquired by the acquisition part 331, the detection part 332 detects a tap event indicating that a person to be analyzed tapped a device (S12). The detection part 332 detects the tap event on condition that a peak of acceleration occurred a predetermined number of times or more within a predetermined time period in the acceleration data, for example.

The estimation part 333 inputs the electrocardiogram data of a first target period within the electrocardiogram data acquired by the acquisition part 331 to a first machine learning model stored in the storage part 32. The estimation part 333 estimates whether atrial fibrillation occurs in the person to be analyzed in the first target period on the basis of a classification result output from the first machine learning model (S13).

The display control part 335 generates display information for displaying information indicating a relationship between the tap event detected by the detection part 332 and the presence or absence of atrial fibrillation estimated by the estimation part 333, together with electrocardiogram data of the first target period, on the display part of the information terminal 2, and transmits the display information to the information terminal 2 (S14). According to the display information received from the display control apparatus 3, the information terminal 2 displays the information indicating the relationship between the tap event detected by the detection part 332 and the presence or absence of atrial fibrillation estimated by the estimation part 333, together with the electrocardiogram data of the first target period, on the display part.

The acquisition part 331 acquires a feature value that is a value indicating electric activity of the heart of the person to be analyzed, from the electrocardiogram data (S15). For example, the acquisition part 331 acquires any one of an R-R interval, a heart rate per unit time, a V-V interval, and a V-A interval as a feature value.

The selection part 334 selects at least a part of the period, during which the measurement of the electric activity of the heart is performed on the person to be analyzed, as a second target period during which the feature value is to be displayed (S16). The selection part 334 selects the second target period on the basis of variation of a plurality of feature values, the tap event detected by the detection part 332, or the presence or absence of atrial fibrillation estimated by the estimation part 333, for example.

The estimation part 333 inputs electrocardiogram data of the second target period within the electrocardiogram data acquired by the acquisition part 331 to the first machine learning model stored in the storage part 32. The estimation part 333 estimates whether atrial fibrillation occurs in the person to be analyzed in the second target period on the basis of a classification result output from the first machine learning model (S17).

The display control part 335 arranges a plurality of points P1 corresponding to a plurality of feature values measured in the second target period on a two-dimensional plane relative to a predetermined reference point (S18). The display control part 335 arranges the plurality of points such that a distance between the reference point and each of the plurality of points corresponds to the feature value corresponding to said point. Further, the display control part 335 arranges the plurality of points such that a direction of the vector from the reference point to each of the plurality of points rotates clockwise or counterclockwise about the reference point in the order of the times at which the plurality of feature values are measured.

The display control part 335 generates display information including the electrocardiogram data of the second target period and information for displaying a polygonal figure, and transmits the generated display information to the information terminal 2 (S19), for example. In accordance with the display information received from the display control apparatus 3, the information terminal 2 displays a polygonal figure corresponding to the feature values on the display part.

In the flowchart illustrated in FIG. 9, processes related to display of the tap event in steps S12 to S 14 and processes related to display of the polygonal figure in steps S15 to S 19 may be performed in parallel, in the reverse order, or only one of them may be performed.

### EFFECTS OF THE EMBODIMENT

In the display control system S according to the present embodiment, the display control apparatus 3 causes the display part of the information terminal 2 to display change over time of the feature values indicating the electric activity of the heart of the person to be analyzed, in the shape of the polygonal figure. The analyzer can grasp change over time of the feature values in the target period as the shape of the polygonal figure in the information terminal 2. For example, the shape of the polygonal figure becomes close to a true circle in the normal state, and unevenness of the overall shape of the polygonal figure increases in the state in which atrial fibrillation occurs in the person to be analyzed. Further, unevenness of only a part of the shape of the polygonal figure increases in the situation in which premature contraction occurs in the person to be analyzed, for example. As a result, the display control system S can make it easier for the analyzer to confirm change over time of the value indicating the electric activity of the heart. Therefore, even if the analyzer is not a specialist, a possibility of occurrence of atrial fibrillation, premature contraction, and a normal waveform can be easily distinguished by referencing the shape of the polygonal figure, for example.

### [First variation example]

The display control apparatus 3 according to the present variation example causes the display part to sequentially display points corresponding to the feature values in response to the feature values being measured. In this variation example, the selection part 334 does not need to select the second target period during which the feature value is to be displayed.

The display control part 335 arranges a point P1 corresponding to new feature value on the two-dimensional plane relative to the reference point P0 according to the rules described with reference to FIG. 6.

On condition that a new point P1 has been arranged, the display control part 335 generates display information for displaying a polygonal figure including the new point P1. The information for displaying the polygonal figure is coordinates of each of a plurality of points P1 including the new point P1 or an image of the plurality of points P1 including the new point P1 arranged in the two-dimensional plane, for example. By doing this, the display control apparatus 3 can sequentially display the plurality of points P1 corresponding to the plurality of feature values in the order of the times at which they are measured, on the display part.

The display control part 335 may delete a point P1 from the polygonal figure on condition that a predetermined time has passed from the time at which the feature value corresponding to this point P1 included in the polygonal figure was measured. By doing this, the display control apparatus 3 causes the display part to display a polygonal figure including only the plurality of points P1 corresponding to the recently measured feature values, and can make it easier for the analyzer to grasp the recent state of the person to be analyzed.

The display control part 335 transmits the generated display information to the information terminal 2. According to the display information received from the display control apparatus 3, the information terminal 2 displays a polygonal figure corresponding to the feature values on the display part.

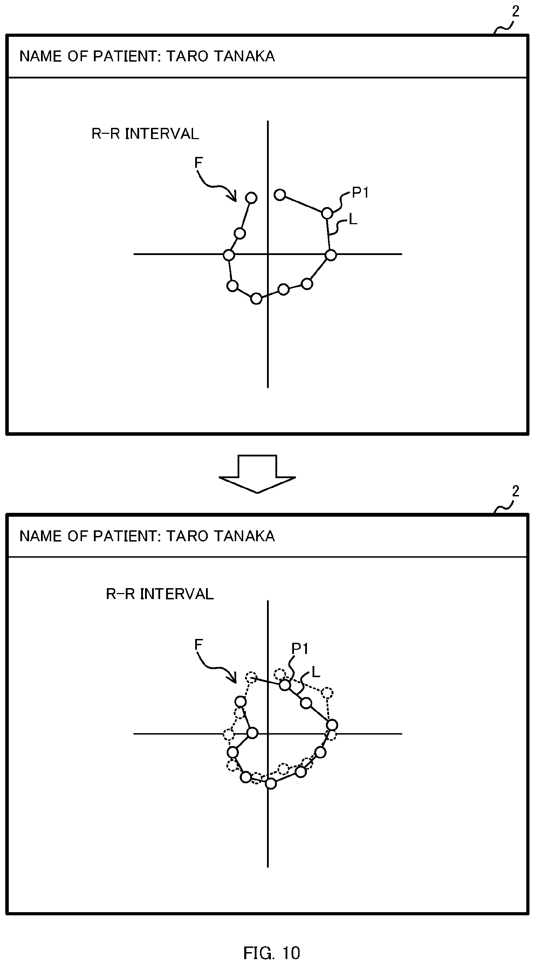

FIG. 10 is a schematic diagram of the information terminal 2 displaying a polygonal figure corresponding to the feature values. In the example of FIG. 10, the display control part 335 causes a polygonal figure F shown in the above diagram to be displayed on the display part of the information terminal 2, and then causes a polygonal figure F shown in the below diagram to be displayed on the display part of the information terminal 2.

The polygonal figure F includes a plurality of points P1 corresponding to a plurality of feature values, and a polygonal line L generated by connecting two adjacent points among the plurality of points P1 with a line. Further, the polygonal figure F may include only one of the plurality of points P1 or the polygonal line L. Further, as in the example of FIG. 8, the polygonal figure F may be displayed in a manner such that it is superimposed on a plurality of concentric circles arranged at predetermined intervals and centered around the reference point used for arranging the plurality of points P1.

The display control part 335 may display the plurality of points P1 included in the polygonal figure F on the display part all at once. Further, the display control part 335 may display the plurality of points P1 included in the polygonal figure F on the display part one by one in the order of the times at which the points P1 are measured.

As an example, the display control part 335 generates the polygonal line L by connecting two points P1 from among the plurality of points P1 in the order of the times at which the feature values are measured. Further, the display control part 335 may connect a point P1 which is measured at the earliest time and a point P1 which is measured at the latest time among the plurality of points P1 with a line. Further, the display control part 335 may generate the polygonal line L by connecting two points P1 in the order of positions (coordinates) close to each other among the plurality of points P1. The polygonal line L may be generated by the information terminal 2 instead of the display control part 335 of the display control apparatus 3. By causing the change over time of the feature values to be displayed in the shape of the polygonal figure in this manner, the display control apparatus 3 can make it easier for the analyzer to confirm the change over time of the feature values.

The display control part 335 may change a display mode of a point P1 corresponding to a feature value or a line connected to said point P1, according to the time at which the feature value is measured. For example, the display control part 335 changes a color, pattern, or size of a plurality of points P1 or changes a color, type, or thickness of a line connected to each of the plurality of points P1 depending on whether (i) a difference between the current time and the time at which the feature value was measured is within a predetermined threshold value or (ii) a difference between the current time and the time at which the feature value was measured is greater than the predetermined threshold value. By doing this, the display control apparatus 3 changes the display mode for an old point P1 and for new point P1 to make it easier for the analyzer to intuitively grasp change over time of the feature values corresponding to the plurality of points P1.

When a plurality of periods constituting the second target period are represented with multiple cycles of the polygonal figure F, the display control part 335 may cause the display part to display a polygonal figure F of a second period within the second target period superimpose on a polygonal figure F of a first period within the second target period. For example, the first period corresponds to a period corresponding to 360 degrees on the two-dimensional plane, and the second period corresponds to a period corresponding to the next 360 degrees on the two-dimensional plane. By doing this, the display control apparatus 3 can display the plurality of points P1 while rotating them about the reference point, and can make it easier for the analyzer to grasp change over time of the shape of the polygonal figure F.

In this case, the display control part 335 may cause the display part to display the polygonal figure F of the first period and the polygonal figure F of the second period as an unclosed polygonal line by connecting them with a line. Further, the display control part 335 may display the polygonal figure F of the first period and the polygonal figure F of the second period on the display part as closed polygonal lines, respectively.

Further, the display control part 335 may multiply the distance between the reference point and each of the plurality of points P1 by a coefficient that increases or decreases over time. Then, even if the feature values are constant, the distance between the reference point and each of the plurality of points P1 changes over time, and therefore the polygonal figure F takes the shape of spiral. As a result, the display control apparatus 3 can make it difficult for the plurality of points P1 to overlap each other when displaying the plurality of points P1 while rotating them.

While the plurality of points are sequentially displayed on the display part, the display control part 335 may stop displaying the plurality of points on the display part sequentially if the variation of the plurality of measured feature values becomes equal to or greater than a predetermined threshold value. In this case, the acquisition part 331 calculates variation of the plurality of feature values that have been measured so far, on condition that a new feature value is acquired. The acquisition part 331 may calculate the variation using the entire plurality of measured feature values, or may calculate the variation using some of the plurality of measured feature values (for example, a predetermined number of feature values retrospective from the latest feature value).

The acquisition part 331 calculates a statistic value such as a variance, standard deviation, or the like of the plurality of feature values as the variation of the plurality of feature values, for example. Further, the acquisition part 331 may calculate a value corresponding to a difference from a mode value or a median value of the plurality of feature values, as the variation of the plurality of feature values.

For example, if the calculated variation becomes equal to or greater than a predetermined threshold value, the display control part 335 stops sequentially arranging new points P1, and transmits display information for displaying a polygonal figure including the plurality of points already arranged to the information terminal 2. By doing this, the display control apparatus 3 stops updating the polygonal figure F at a timing when the variation of the plurality of feature values increases, and therefore it can prevent a portion important for confirming whether atrial fibrillation occurs from being overwritten with subsequent data.

### [Second variation example]

Depending on the trends of change over time of the feature values, the period corresponding to one cycle of the polygonal figure, i.e., the length of the period corresponding to one cycle, may vary. Therefore, the display control system S according to the present variation example makes the period corresponding to one cycle of the polygonal figure to be displayed on the information terminal 2 switchable. Hereinafter, differences from the above-described embodiment will be mainly described.

The acquisition part 331 acquires a period corresponding to one cycle of the polygonal figure to be displayed on the information terminal 2, the period being designated by the analyzer in the information terminal 2. The period corresponding to one cycle is represented by a measurement duration of electrocardiogram data such as 10 seconds, 30 seconds, or the like, or the number of vertices of a polygonal figure such as 10 vertices, 30 vertices, or the like, for example.

For example, the display control part 335 determines the angle θ at which the vector V of the point P1 corresponding to the feature value is rotated as illustrated in FIG. 6, so that the polygonal figure is displayed in the designated period of one cycle. The display control part 335 arranges a plurality of points P1 corresponding to a plurality of feature values on a two-dimensional plane relative to a predetermined reference point P0 (origin) using the determined angle θ. By doing this, the plurality of feature values are displayed as the polygonal figure that corresponds to the period of the one cycle designated by the analyzer.

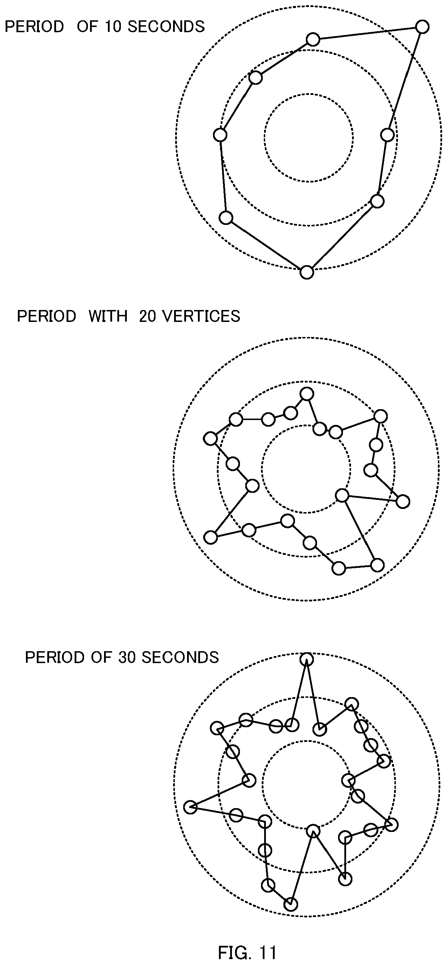

FIG. 11 is a schematic diagram for illustrating the shape of an exemplary polygonal figure. The example of FIG. 11 represents a polygonal figure displayed on the basis of electrocardiogram data in a case where atrial fibrillation and bradycardia occur in a person to be analyzed. Since the heart rate is low in a case of bradycardia, it is difficult for the analyzer to confirm unevenness due to atrial fibrillation in a polygonal figure whose period corresponding to one cycle is 10 seconds. On the other hand, in a polygonal figure whose period of for one cycle is 20 vertices or 30 seconds, the analyzer can easily confirm unevenness caused by atrial fibrillation.

By making the period corresponding to one cycle of the polygonal figure to be displayed on the information terminal 2 switchable in this manner, the display control system S according to the present variation example can make it easier to confirm the trends of change over time of the feature values.

### [Third variation example]

In the above-described embodiment, an example has been described in which the shape of the polygonal figure is used to determine whether atrial fibrillation or premature contraction occurs in the person to be analyzed, but the determination is not limited to the occurrence of atrial fibrillation or premature contraction, and the shape of the polygonal figure can be used for determining whether other arrhythmias have occurred as well.

As described above, the polygonal figure has a shape with large unevenness overall in the state in which atrial fibrillation occurs in the person to be analyzed. In the state in which premature contraction occurs in the person to be analyzed, the polygonal figure has large unevenness in one portion and a shape approximate to an arc in the other portion. In a state where bigeminy or trigeminy occurs in a person to be analyzed, the polygonal figure takes the shape of a star.

In a state where tachycardia occurs in a person to be analyzed, the polygonal figure has a shape indicating that the R-R intervals, i.e., feature values, are small or the heart rate per unit time is high (e.g., a shape having a relatively small area). In a state where bradycardia occurs in a person to be analyzed, the polygonal figure has a shape indicating that the R-R intervals, i.e., feature values, are large or the heart rate per unit time is low (e.g., a shape having a relatively large area). In a state in which a pause (stop of a heartbeat) occurs in a person to be analyzed, the polygonal figure has a shape indicating that the R-R intervals, i.e., feature values, are large or the heart rate per unit time is low (e.g., a shape having a relatively large area).

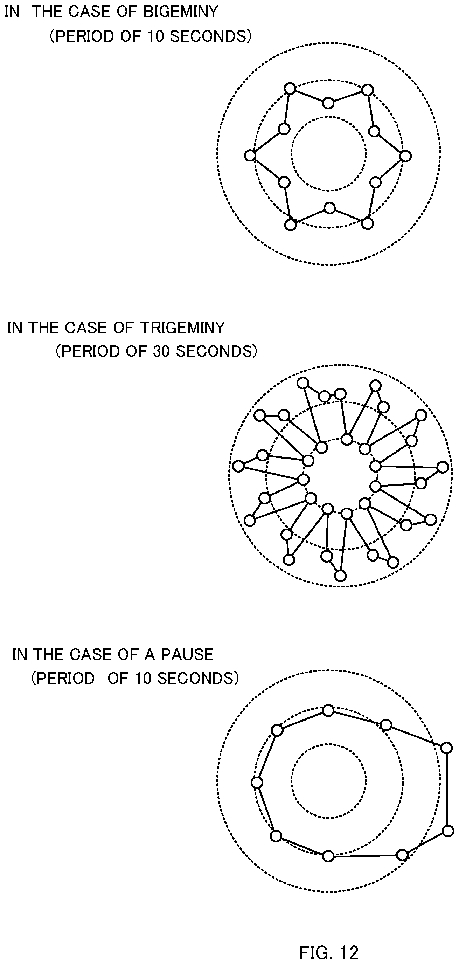

FIG. 12 is a schematic diagram for illustrating the shape of an exemplary polygonal figure. The example of FIG. 12 represents a polygonal figure displayed on the basis of electrocardiogram data in a case where bigeminy, trigeminy, or a pause occurs in a person to be analyzed. The polygonal figure in a case where bigeminy occurs takes the shape of a star. The polygonal figure in a case where trigeminy occurs has a shape in which vertex portions of the star shape are bent downward. The polygonal figure in a case where a pause occurs has a shape in which vertices of a certain portion are abnormally larger than the vertices of the remaining portion.

By displaying the polygonal figure on the information terminal 2 in this manner, the display control system S can make it easier for the analyzer to determine whether various arrhythmias including atrial fibrillation or premature contraction occur in the person to be analyzed.

It is desirable that the polygonal figure is displayed in a manner such that it is superimposed on an auxiliary line which is a circle having a predetermined reference value as a radius and centered around the reference point P0 used for arranging the plurality of points P1.The reference value of the auxiliary line is an R-R interval or the heart rate per unit time indicating that tachycardia occurs, an R-R interval or the heart rate per unit time indicating that bradycardia occurs, an R-R interval or the heart rate per unit time indicating that a pause occurs, or the like. By comparing the polygonal figure and the auxiliary line in this manner, the display control system S can make it easier for the analyzer to determine whether tachycardia, bradycardia, pause, or the like occurs in the person to be analyzed.

### [Fourth variation example]

At about the point in time when atrial fibrillation starts or ends in a person to be analyzed, a portion of atrial fibrillation and a portion of sinus rhythm are mixed in one polygonal figure, so that it is difficult to distinguish between atrial fibrillation and premature contraction. Therefore, the display control system S according to the present variation example generates a polygonal figure of a period in which atrial fibrillation occurs over the entire range. Hereinafter, differences from the above-described embodiment will be mainly described.

The estimation part 333 estimates whether atrial fibrillation occurs in the person to be analyzed for each of a plurality of pieces of electrocardiogram data generated by dividing the entire period of the electrocardiogram data acquired by the acquisition part 331 into predetermined lengths (for example, a length shorter than the period corresponding to one cycle of the polygonal figure) using the first machine learning model described above. The display control part 335 causes the information terminal 2 to display the polygonal figure using feature values corresponding to a plurality of pieces of consecutive electrocardiogram data (electrocardiogram data of a period in which it is estimated that atrial fibrillation occurs over the entire range) determined to have occurred, for example.

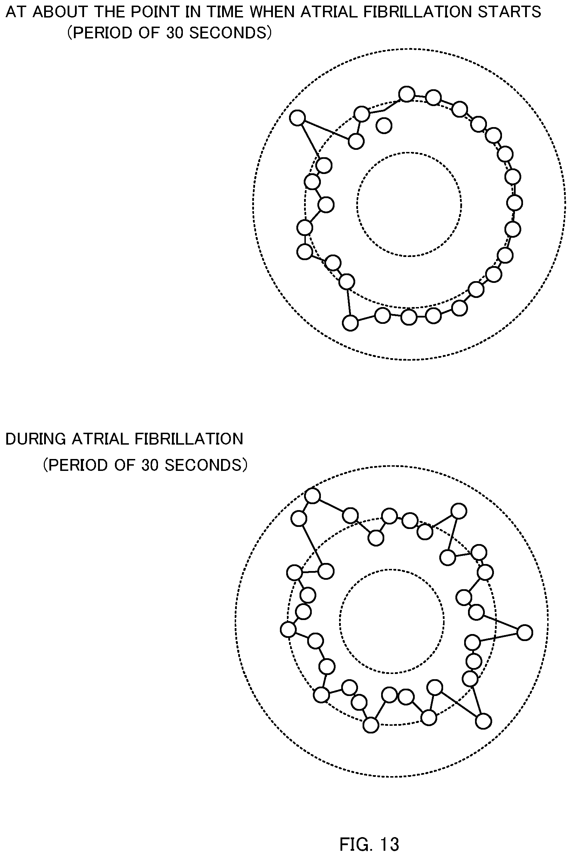

FIG. 13 is a schematic diagram for illustrating the shape of an exemplary polygonal figure. The example in the upper part of FIG. 13 represents a polygonal figure corresponding to electrocardiogram data at about the point in time when atrial fibrillation starts in the person to be analyzed. At about the point in time when atrial fibrillation starts in the person to be analyzed, a shape having large unevenness and a shape approximate to an arc are mixed in the polygonal figure, and therefore it is difficult to distinguish the shape approximate to the arc of the polygonal figure when premature contraction occurs in the person to be analyzed. The same applies at about the point in time when atrial fibrillation ends.

The example in the lower part of FIG. 13 represents a polygon corresponding to electrocardiogram data of a period in which it is estimated that atrial fibrillation occurs over the entire range in the person to be analyzed. In the period in which atrial fibrillation occurs over the entire range, e.g., during the period in which atrial fibrillation is occurring in the person to be analyzed, the analyzer can easily determine that atrial fibrillation occurs in the person to be analyzed since the entire polygonal figure takes a shape having large unevenness.

As described above, in the display control system S according to the present variation example, by causing the information terminal 2 to display the polygonal figure of the period in which atrial fibrillation occurs over the entire range, the analyzer can easily determine the presence or absence of atrial fibrillation or premature contraction by referencing the polygonal figure.

The present disclosure is explained based on the exemplary embodiments. The technical scope of the present disclosure is not limited to the scope explained in the above embodiments and it is possible to make various changes and modifications within the scope of the disclosure. For example, all or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments.

The processor of the display control apparatus 3 performs each step (step) included in the display control method shown in FIG. 9. That is, the processor of the display control apparatus 3 executes the display control method shown in FIG. 9 by executing a program for executing the display control method shown in FIG. 9 to control each unit of the display control system S. The steps included in the display control method shown in FIG. 9 may be partially omitted, the order of the steps may be changed, or a plurality of steps may be performed in parallel.

### [Description of the reference numerals]

S Display control system
1 Measurement equipment
11 Communication part
12 Electrocardiogram measurement part
13 Acceleration measurement part
2 Information terminal
3 Display control apparatus
31 Communication part
32 Storage part
33 Control part
331 Acquisition part
332 Detection part
333 Estimation part
334 Selection part
335 Display control part

## Claims

1. A display control apparatus, comprising:
an acquisition part that acquires a value indicating electric activity of the heart of a person to be analyzed; and
a display control part that causes a display part to display a plurality of points corresponding to a plurality of values measured in a target period, wherein
a distance between a predetermined reference point and each of the plurality of points corresponds to the value corresponding to the point, and
a direction of a vector from the reference point to each of the plurality of points rotates clockwise or counterclockwise about the reference point in the order of times at which the plurality of values are measured.

2. The display control apparatus according to claim 1, wherein
the display control part causes the display part to display a polygonal line generated by connecting two adjacent points among the plurality of points with a line.

3. The display control apparatus according to claim 1 or 2, wherein
the display control part changes a display mode of a point corresponding to a value or a line connected to the point, according to the time at which the value is measured.

4. The display control apparatus according to claim 1 or 2, wherein
the display control part causes the display part to display a plurality of concentric circles arranged at predetermined intervals centered around the reference point together with the plurality of points.

5. The display control apparatus according to claim 1 or 2, further comprising:
a selection part that selects, as the target period, a period in which variation of the plurality of values satisfies a predetermined condition from within a period during which measurement of the electric activity is performed.

6. The display control apparatus according to claim 1 or 2, further comprising:
a selection part that selects, as the target period, a period during which it is estimated that atrial fibrillation or premature contraction occurs in the person to be analyzed from a period during which a measurement of the electric activity is performed.

7. The display control apparatus according to claim 6, further comprising:
an estimation part that estimates whether atrial fibrillation or premature contraction occurs in the person to be analyzed by inputting a positional relationship among the plurality of points corresponding to the plurality of values of the person to be analyzed to a machine learning model that is machine-learned using (i) a positional relationship among a plurality of points corresponding to a plurality of values in a period in which it is determined that atrial fibrillation or premature contraction is occurring in a person to be learned, and (ii) a positional relationship among a plurality of points corresponding to a plurality of values in a period in which it is determined that atrial fibrillation or premature contraction is not occurring in the person to be learned.

8. The display control apparatus according to claim 6, wherein
the display control part causes the display part to display (i) the plurality of points in the target period in which it is estimated that atrial fibrillation or premature contraction occurs in the person to be analyzed and (ii) the plurality of points in the target period in which it is estimated that atrial fibrillation or premature contraction does not occur in the person to be analyzed, in different display modes.

9. The display control apparatus according to claim 1 or 2, wherein
the acquisition part calculates circularity that becomes smaller as a shape of a polygonal line generated by connecting two adjacent points among the plurality of points is closer to a true circle, and
the display control part causes the display part to display the plurality of points whose circularity is equal to or less than a threshold value and the plurality of points whose circularity is greater than the threshold value in different display modes.

10. The display control apparatus according to claim 9, wherein
the longer the target period or greater the number of the plurality of points, the smaller the threshold value is.

11. The display control apparatus according to claim 1 or 2, wherein
the display control part causes the display part to display the plurality of points in a second period within the target period in a manner such that the plurality of points in the second period are superimposed on the plurality of points in a first period within the target period.

12. The display control apparatus according to claim 1 or 2, wherein
an angle between two points relative to the reference point is an angle proportional to the value corresponding to one of the two points.

13. The display control apparatus according to claim 1 or 2, wherein
the display control part causes the display part to sequentially display the points corresponding to the values in response to the values being measured.

14. The display control apparatus according to claim 13, wherein
the display control part stops displaying the points corresponding to the values on the display part sequentially if variation of the plurality of values becomes equal to or greater than a predetermined threshold value while the points corresponding to the values are sequentially displayed on the display part.

15. The display control apparatus according to claim 1 or 2, further comprising:
a detection part that detects a tap event indicating that the person to be analyzed tapped a device for measuring the electric activity, on the basis of acceleration measured by a sensor provided in the device, the device being worn by the person to be analyzed; and
a selection part that selects, as the target period, a period based on a time at which the tap event is detected from a period during which the electric activity is measured.

16. The display control apparatus according to claim 15, further comprising:
an estimation part that estimates whether atrial fibrillation occurs in the person to be analyzed on the basis of the electric activity measured during a period based on a time at which the tap event is detected, wherein
the display control part causes the display part to display information indicating whether atrial fibrillation estimated by the estimation part occurs in the person to be analyzed.

17. The display control apparatus according to claim 15, wherein
the detection part detects the tap event on condition that the sensor measures acceleration equal to or greater than a threshold value which is greater than acceleration generated in the sensor by the electric activity.

18. A display control method executed by a processor and comprising the steps of:
acquiring a value indicating electric activity of the heart of a person to be analyzed; and
causing a display part to display a plurality of points corresponding to a plurality of values measured in a target period, wherein
a distance between a predetermined reference point and each of the plurality of points corresponds to the value corresponding to the point, and
a direction of a vector from the reference point to each of the plurality of points rotates clockwise or counterclockwise about the reference point in the order of times at which the plurality of values are measured.

19. A program that causes a computer to function as:
an acquisition part that acquires a value indicating electric activity of the heart of a person to be analyzed; and
a display control part that causes a display part to display a plurality of points corresponding to a plurality of values measured in a target period, wherein
a distance between a predetermined reference point and each of the plurality of points corresponds to the value corresponding to the point, and
a direction of a vector from the reference point to each of the plurality of points rotates clockwise or counterclockwise about the reference point in the order of times at which the plurality of values are measured.
